# EUROPEAN PATENT SPECIFICATION

(11) **EP 4 545 124 B1**
(45) Date of publication and mention of the grant of the patent: **04.02.2026**
(21) Application number: 24737659.3
(22) Date of filing: 23.04.2024
(51) Int. Cl.: G16H 20/40, G16H 40/63, G16H 50/70, G16H 50/30, A61M 16/00, A61H 31/00, G09B 23/28

(54) **VENTILATOR CONFIGURED FOR IMPLEMENTING A METHOD BASED ON AUTOMATIC AIRWAY PRESSURE FEEDBACK FOR CARDIOPULMONARY RESUSCITATION**
BEATMUNGSGERÄT ZUR IMPLEMENTIERUNG EINES VERFAHRENS ZUR AUTOMATISCHEN FEEDBACKBEATMUNG DES ATEMWEGSDRUCKS ZUR KARDIOPULMONALEN REANIMATION
VENTILATEUR CONFIGURÉ POUR METTRE EN OEUVRE UNE MÉTHODE DE VENTILATION BASÉE SUR UNE RÉTROACTION AUTOMATIQUE DE PRESSION DES VOIES RESPIRATOIRES POUR LA RÉANIMATION CARDIO-PULMONAIRE

(30) Priority: 14.09.2023 CN 202311189234
(43) Date of publication of application: 30.04.2025
(73) Proprietor: Guangzhou Landswick Medical Technologies Ltd., Guangzhou, Guangdong 510000 (CN)
(72) Inventor: SUN, Caixin, Guangzhou, Guangdong 510000 (CN); LIN, Shengxiang, Guangzhou, Guangdong 510000 (CN); DONG, Hui, Guangzhou, Guangdong 510000 (CN)
(74) Representative: Santarelli
(86) International application number: PCT/CN2024/089230
(87) International publication number: WO 2025/055334

(56) References cited:
- CN-A- 105 664 313
- CN-A- 107 296 737
- CN-A- 117 159 862
- CN-U- 212 369 272
- US-A1- 2013 085 425
- JAUREGUIBEITIA XABIER ET AL: "Automatic Detection of Ventilations During Mechanical Cardiopulmonary Resuscitation", IEEE JOURNAL OF BIOMEDICAL AND HEALTH INFORMATICS, IEEE, PISCATAWAY, NJ, USA, vol. 24, no. 9, 16 January 2020 (2020-01-16), pages 2580 - 2588, XP011807154, ISSN: 2168-2194, [retrieved on 20200903], DOI: 10.1109/JBHI.2020.2967643
- ALI JALALI ET AL: "Model based optimization of the cardiopulmonary resuscitation (CPR) procedure", ENGINEERING IN MEDICINE AND BIOLOGY SOCIETY (EMBC), 2013 34TH ANNUAL INTERNATIONAL CONFERENCE OF THE IEEE, IEEE, 28 August 2012 (2012-08-28), pages 715 - 718, XP032463018, ISSN: 1557-170X, DOI: 10.1109/EMBC.2012.6346031

## Description

### Technical Field

The present disclosure relates to the field of medical emergency technology, particularly to a ventilator configured for carrying out a cardiopulmonary resuscitation airway pressure automatic feedback ventilation method.

### Background Art

Currently, cardiopulmonary resuscitation (CPR) is the basic and the foremost medical method for rescuing patients with cardiac arrest, with external chest compressions combined with forced ventilation often being the emergency measures that such patients require promptly to maintain basic blood circulation, heartbeat, and respiratory recovery. Existing cardiopulmonary resuscitation typically includes two major components: cardiac compressions and ventilation (breathing); cardiac compressions usually have two forms, manual and mechanical (equipment), with manual compressions used when conditions do not permit, and mechanical (equipment) compressions used in hospitals when conditions allow. Clinical requirements for compressions usually follow: effective external chest compression frequency at 100~120 times/minute, depth at 5~6 centimeters; ventilation (breathing) during cardiopulmonary resuscitation emergency is usually performed using: manual blowing, bag-valve-mask ventilation, and ventilator ventilation. Ventilation requirements according to clinical demands include 2 ventilations in the middle of 30 compressions, 2 ventilations after 15 compressions, and continuous uninterrupted compressions with 1 ventilation every 10 compressions. The coordination of existing cardiopulmonary resuscitation compressions and ventilation is usually managed by medical staff or by the coordination of cardiopulmonary resuscitation compression equipment and ventilators through communication protocols. There are issues with the inability to perform automatic coordination and the difficulty of manual coordination methods to precisely and stably control for long periods, reducing practicality.

Xabier Jaureguibeitia et al. (Jaureguibeitia, Xabier, et al. "Automatic detection of ventilations during mechanical cardiopulmonary resuscitation." IEEE journal of biomedical and health informatics 24.9 (2020): 2580-2588.) introduce a method for accurate ventilation detection using the impedance while chest compressions are concurrently delivered by a mechanical CPR device. The method efficiently combines adaptive signal processing techniques to obtain and detect ventilation waveforms, with a machine learning algorithm to identify true ventilations. The study also evaluated the performance of the model to detect ventilations and measure ventilation rate.

### Summary of the Invention

The invention is set out in the appended claims.

In view of the problems presented above, the present disclosure provides a ventilator to solve the issues mentioned in the background art regarding the coordination of existing cardiopulmonary resuscitation compressions and ventilation, which is usually managed by medical staff or through communication protocols between cardiopulmonary resuscitation compression equipment and ventilators. The inability to perform automatic coordination and the difficulty of manual coordination methods to precisely and stably control for long periods, reducing practicality. The references to the methods of treatment by therapy in this description are to be interpreted as references to the ventilator of the present invention configured for carrying out those methods.

Other features and advantages of this invention will be described in the subsequent specification, and will, in part, be apparent from the specification, or may be learned by practice of the invention. The objectives and other advantages of the present disclosure may be realized and obtained by means of the structures particularly pointed out in the written specification and drawings.

The following detailed description of the embodiments of the present disclosure will be made with reference to the accompanying drawings.

### Description of the Drawings

The drawings are intended to provide further understanding of the present disclosure and constitute a part of this specification. They illustrate embodiments of the present disclosure and, together with the description, serve to explain the principles of the disclosure and are not intended to limit the scope of the disclosure.
FIG. 1 is a workflow diagram of a cardiopulmonary resuscitation airway pressure automatic feedback ventilation method provided by the present disclosure;
FIG. 2 is another workflow diagram of a cardiopulmonary resuscitation airway pressure automatic feedback ventilation method provided by the present disclosure;
FIG. 3 is yet another workflow diagram of a cardiopulmonary resuscitation airway pressure automatic feedback ventilation method provided by the present disclosure;
FIG. 4 is a schematic structural diagram of a ventilator provided by the present disclosure;
FIG. 5 is a screenshot of an embodiment generated according to a cardiopulmonary resuscitation airway pressure automatic feedback ventilation method provided by the present disclosure;
FIG. 6 is a curve of airway pressure during the cardiac compression process;
FIG. 7 is a schematic diagram for signal preprocessing;
FIG. 8 is a schematic diagram for frequency domain analysis of the signal;
FIG. 9 is a schematic diagram for time domain analysis of the signal;
FIG. 10 is a schematic diagram for determining the timing of ventilation using a dynamic threshold method on machine compression signals;
FIG. 11 is a schematic diagram for determining the timing of ventilation using a dynamic threshold method on manual compression signals.

### Detailed Description of Embodiments

The exemplary embodiments will be described in detail here, with examples illustrated in the accompanying drawings. When the following description refers to the drawings, unless otherwise indicated, the same numbers in different drawings refer to the same or similar elements. The embodiments described in the following exemplary embodiments do not represent all embodiments consistent with the present disclosure. Instead, they are merely examples of devices and methods consistent with some aspects of the disclosure as detailed in the appended claims.

Currently, cardiopulmonary resuscitation (CPR) is the basic and foremost medical method for rescuing patients with cardiac arrest. External chest compressions combined with forced ventilation are often the emergency measures that such patients require promptly to maintain basic blood circulation, heartbeat, and respiratory recovery. Existing cardiopulmonary resuscitation typically includes two major components: cardiac compressions and ventilation (breathing); cardiac compressions usually have two forms, manual and mechanical (equipment), with manual compressions used when conditions do not permit, and mechanical (equipment) compressions used in hospitals when conditions allow. Clinical requirements for compressions usually follow: effective external chest compression frequency at 100~120 times/minute, depth at 5~6 centimeters; ventilation (breathing) during cardiopulmonary resuscitation emergency is usually performed using: manual blowing, bag-valve-mask ventilation, and ventilator ventilation. Ventilation requirements according to clinical demands include 2 ventilations in the middle of 30 compressions, 2 ventilations after 15 compressions, and continuous uninterrupted compressions with 1 ventilation every 10 compressions. The coordination of existing cardiopulmonary resuscitation compressions and ventilation is usually managed by medical staff or by the coordination of cardiopulmonary resuscitation compression equipment and ventilators through communication protocols. There are issues with the inability to perform automatic coordination and the difficulty of manual coordination methods to precisely and stably control for long periods, reducing practicality. To solve the aforementioned problems, this embodiment discloses a cardiopulmonary resuscitation airway pressure automatic feedback ventilation method.

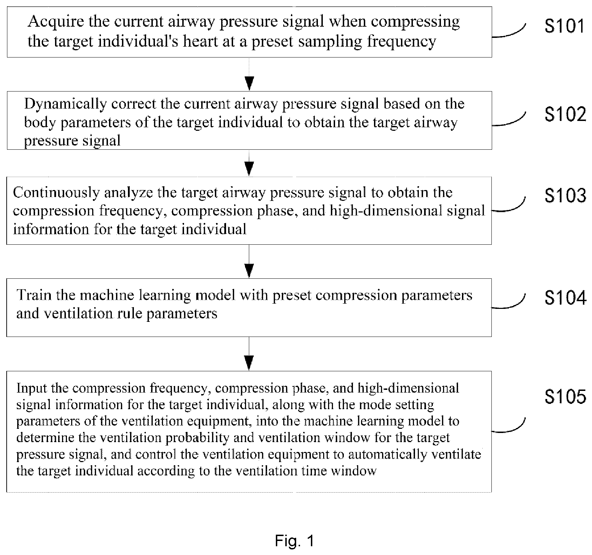

A cardiopulmonary resuscitation airway pressure automatic feedback ventilation method, as shown in FIG. 1, includes the following steps:
Step S101, obtaining the current airway pressure signal when compressing the heart of the target individual at a preset sampling frequency;
Step S102, dynamically correcting the current airway pressure signal based on the body parameters of the target individual to obtain the target airway pressure signal;
Step S103, continuously analyzing the target airway pressure signal to obtain the compression frequency, compression phase, and high-dimensional signal information for the target individual;
Step S104, training a machine learning model with preset compression parameters and ventilation rule parameters;
Step S105, inputting the compression frequency, compression phase, and high-dimensional signal information for the target individual, along with the mode setting parameters of the ventilation equipment, into the machine learning model to determine the target pressure signal's ventilation probability and ventilation time window, and controlling the ventilation equipment to perform automatic ventilation on the target individual according to the ventilation time window.

In this embodiment, the preset sampling frequency refers to the collection cycle frequency of the sampling signal;

In this embodiment, body parameters refer to the target individual's body shape description parameters, such as height, weight, and specific indices;

In this embodiment, dynamic correction refers to correcting the current airway pressure signal based on the impact factor of the target individual's body shape on the compression force;

In this embodiment, continuous analysis refers to the continuous analysis of the target airway pressure signal in terms of time and frequency;

In this embodiment, the compression frequency, compression phase, and high-dimensional signal information for the target individual refer to the frequency and amplitude of chest compressions on the target individual;

In this embodiment, preset compression parameters and ventilation rule parameters refer to the preset rules correlating the number of compressions with the timing of ventilation, for example, one ventilation after every 30 compressions;

In this embodiment, the mode setting parameters of the ventilation equipment refer to the ventilation setting parameters of the ventilation equipment in various working modes;

In this embodiment, ventilation probability and ventilation time window refer to the probability of the user receiving ventilation with each chest compression and the description duration window for confirming the number of chest compressions for ventilation.

The working principle of the above technical solution is as follows: obtain the current airway pressure signal when compressing the heart of the target individual at a preset sampling frequency; dynamically correct the current airway pressure signal based on the body parameters of the target individual to obtain the target airway pressure signal; continuously analyze the target airway pressure signal to obtain the compression frequency, compression phase, and high-dimensional signal information for the target individual; train a machine learning model with preset compression parameters and ventilation rule parameters; input the compression frequency, compression phase, and high-dimensional signal information for the target individual, along with the mode setting parameters of the ventilation equipment, into the machine learning model to determine the target pressure signal's ventilation probability and ventilation time window, and control the ventilation equipment to perform automatic ventilation on the target individual according to the ventilation time window.

The beneficial effects of the above technical solution are: by automatically predicting the ventilation time window and performing automatic ventilation based on the compression frequency and compression phase of the target individual, the device can automatically perform cardiac compressions and timely ventilation on the target individual without the prerequisite of communication protocols. This reduces labor costs while also precisely controlling the timing of ventilation, improving safety, reliability, and stability. It avoids the influence of subjective factors from medical personnel, enhances objectivity, and solves the problem of existing cardiopulmonary resuscitation compression and ventilation coordination, which is usually managed by medical staff or through communication protocols between cardiopulmonary resuscitation compression equipment and ventilators. The inability to perform automatic coordination and the difficulty of manual coordination methods to precisely and stably control for long periods reduce practicality.

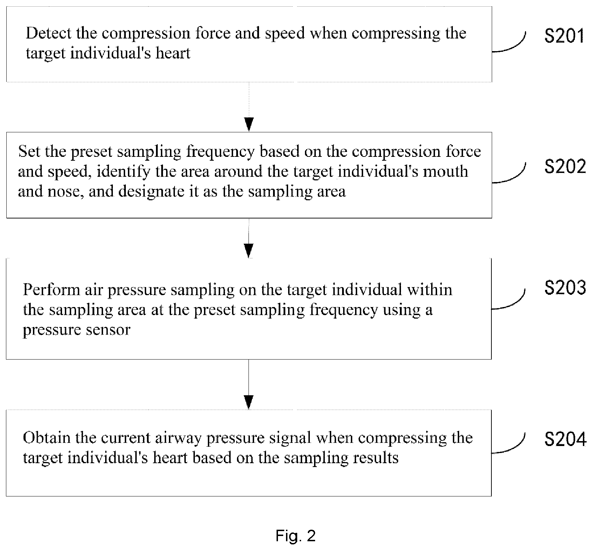

In an embodiment, as shown in Figure 2, the dynamic correction of the current airway pressure signal based on the body parameters of the target individual to obtain the target airway pressure signal includes:
Step S201, detecting the compression force and speed when compressing the heart of the target individual;
Step S202, setting the preset sampling frequency based on the compression force and speed, identifying the area around the mouth and nose of the target individual as the sampling area;
Step S203, performing air pressure sampling on the target individual within the sampling area at the preset sampling frequency using a pressure sensor;
Step S204, obtaining the current airway pressure signal when compressing the heart of the target individual based on the sampling results.

In this embodiment, the sampling area refers to the range area for signal sampling of the current airway pressure signal.

The beneficial effects of the above technical solution are: by intelligently setting the sampling frequency, the sampling frequency can be reasonably set according to the real-time compression situation of the target individual, ensuring the high quality of the sampling data. Furthermore, by locating the sampling area, airflow models and air pressure signals can be more accurately detected around the mouth and nose of the target individual, ensuring the precision of the detected signals.

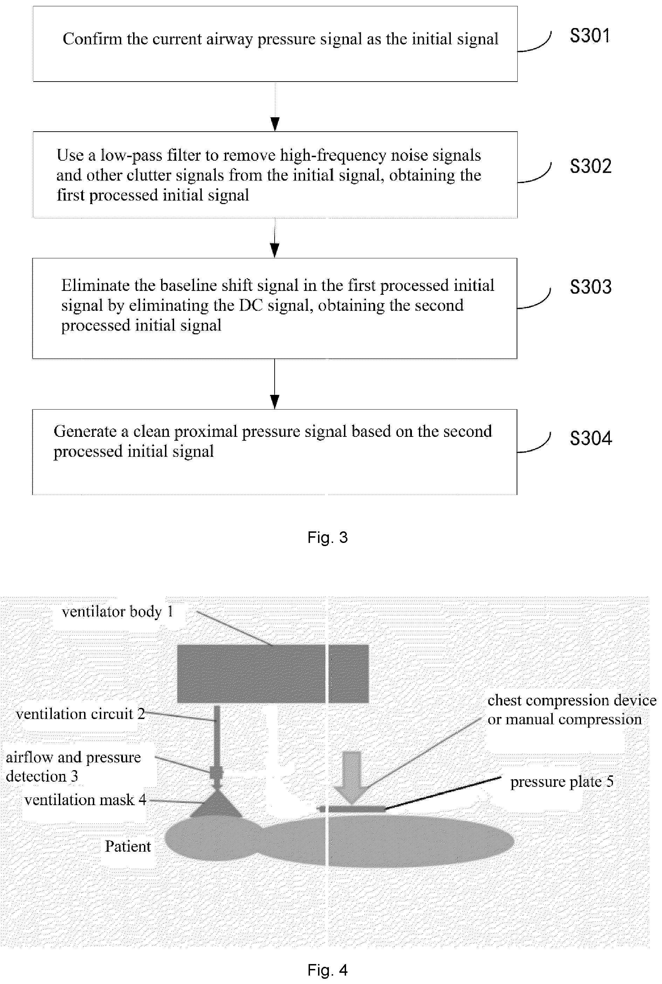

In an embodiment, as shown in Figure 3, before continuously analyzing the current airway pressure signal to obtain the compression frequency, compression phase, and high-dimensional signal information for the target individual, it further includes:
Step S301, confirming the current airway pressure signal as the initial signal;
Step S302, using a low-pass filter to remove high-frequency noise signals and other spurious signals from the initial signal, obtaining the first processed initial signal;
Step S303, eliminating the baseline shift signal from the first processed initial signal by removing the direct current signal, obtaining the second processed initial signal;
Step S304, generating a clean proximal pressure signal based on the second processed initial signal.

In this embodiment, the baseline shift signal refers to the target signal in the first processed initial signal where the signal baseline has shifted.

The beneficial effects of the above technical solution are: it can ensure the purity of the signal and remove the influence of interference signals, laying the foundation for subsequent signal analysis and improving practicality.

In an embodiment, the dynamic correction of the current airway pressure signal based on the body parameters of the target individual to obtain the target airway pressure signal includes:
Determining the current thoracic resonance parameters of the target individual based on their body parameters;
Determining the dynamic correction factor based on the current thoracic resonance parameters and the preset collection parameters of the current airway pressure signal;
Using the dynamic correction factor to dynamically correct the current airway pressure signal;
Obtaining the target airway pressure signal based on the correction results.

In this embodiment, the current thoracic resonance parameters refer to the resonance description parameters between the chest cavity and the compression operation caused by the body shape of the target individual;

In this embodiment, the preset collection parameters refer to the collection frequency and collection intensity settings of the current airway pressure signal;

In this embodiment, the dynamic correction factor refers to the correction factor for the signal phase and signal frequency of the current airway pressure signal.

The beneficial effects of the above technical solution are: by determining the current thoracic resonance parameters based on the body parameters of the target individual and then correcting the collected airway pressure signal, the corrected airway pressure signal is more consistent with the target individual, improving the data reference value.

In this embodiment, determining the dynamic correction factor based on the current thoracic resonance parameters and the preset collection parameters of the current airway pressure signal includes:
Determining the set chest compression variation amplitude parameters based on the preset collection parameters of the current airway pressure signal;
Determining the airway resistance index based on the chest compression variation amplitude parameters and the current thoracic resonance parameters;
Generating a respiratory signal sample set using a respiratory mechanics model based on the airway resistance index;
Determining the morphological parameter values at various points in the target individual's airway based on the respiratory signal sample set;
Determining the range of the stress tensor in the target individual's airway based on the morphological parameter values at various points in the airway;
Acquiring characteristic parameters that maintain the steady-state performance of the target individual's airway, and obtaining the airway performance indicators of the target individual based on the characteristic parameters;
Determining the stress differentiation in the target individual's airway based on the airway performance indicators and the range of the airway's stress tensor;
Determining the standard data for the target individual's chest compression force and the error range of actual detection data based on the stress differentiation;
Determining the dynamic correction factor for the current airway pressure signal based on the standard data for the target individual's chest compression force, the error range of actual detection data, and the detected pressure data corresponding to the current airway pressure signal.

In this embodiment, the set chest compression variation amplitude parameters refer to the amplitude data parameters of the chest cavity fluctuations with compression under set conditions for the target individual;

In this embodiment, the airway resistance index refers to the index of ventilation resistance difficulty in the airway of the target individual under body shape compression;

In this embodiment, the respiratory mechanics model is represented as a network model used for simulating respiratory mechanics;

In this embodiment, the respiratory signal sample set represents a dataset of respiratory signal samples simulated under the airway resistance index through the respiratory mechanics model;

In this embodiment, the morphological parameter values at various points in the target individual's airway represent the parameter values of the undulating morphology at each distributed sampling point in the target individual's airway;

In this embodiment, the range of the stress tensor represents the numerical range of the tensor in the target individual's airway after being subjected to compression force;

In this embodiment, the characteristic parameters that maintain the steady-state performance of the target individual's airway represent the relevant characteristic parameters used to maintain the steady-state performance of the target individual's airway;

In this embodiment, the airway performance indicators represent the indicators for maintaining good respiratory performance of the airway;

In this embodiment, the stress differentiation refers to the differentiation and loss of feedback airflow in the target individual's airway when undergoing chest compressions.

The beneficial effects of the above technical solution are: by determining the airway obstruction parameters of the target individual and then determining the stress differentiation of the target individual's airway during compression, the conversion relationship between the actual compression force and the stress force can be accurately assessed to determine the dynamic correction factor for the detection signal, ensuring the accuracy and objectivity of the final factor.

In an embodiment, continuously analyzing the current airway pressure signal to obtain the compression frequency, compression phase, and high-dimensional signal information for the target individual includes:
Analyzing the proximal pressure signal using a time-domain analysis algorithm to obtain the first analysis result;
Analyzing the proximal pressure signal using a frequency-domain analysis algorithm to obtain the second analysis result;
Determining the compression frequency, compression interval, and duration of a single compression for the target individual based on the first analysis result, and determining the compression phase based on the compression interval and duration of a single compression;
Obtaining the frequency-domain characteristics of the signal based on the second analysis result, and performing a deep analysis of the signal frequency-domain characteristics to obtain high-dimensional signal information.

The beneficial effects of the above technical solution are: by performing dual analysis, the detailed high-dimensional information such as the compression-related rule parameters and compression intensity for the target individual can be accurately analyzed, laying the foundation for subsequent model training and further improving practicality.

In an embodiment, analyzing the proximal pressure signal using a frequency-domain analysis algorithm to obtain the second analysis result includes:
Using a window function and Fourier transform on the proximal pressure signal to perform a time-frequency conversion to obtain a spectral signal;
Marking the peak of the spectral signal, capturing the area around the peak, as well as the areas of the low-frequency and high-frequency regions;
Obtaining the regional signals corresponding to the area around the peak, and the areas of the low-frequency and high-frequency regions;
Performing geometric calculations on the regional signals corresponding to the area around the peak, and the areas of the low-frequency and high-frequency regions using a frequency-domain analysis algorithm to obtain the second analysis result.

The beneficial effects of the above technical solution are: it maximizes the acquisition of the frequency-domain analysis results of the signal, ensuring the completeness and reliability of the high-dimensional information.

In an embodiment, training the machine learning model with preset compression parameters and ventilation rule parameters includes:
Generating compression pattern parameters based on a preset machine learning algorithm according to preset compression parameters;
Generating the first training sample based on the compression pattern parameters, setting model learning parameters and model output parameters based on ventilation rule parameters;
Training the machine learning model with the first training sample based on the model learning parameters and model output parameters.

The beneficial effects of the above technical solution are: by generating training samples and simultaneously setting model learning parameters and output parameters, stable convergence can be achieved during the model training process, ensuring the subsequent stability of model recognition, and improving practicality and reliability.

In an embodiment, inputting the target individual's compression frequency, compression phase, and high-dimensional signal information, along with the mode setting parameters of the ventilation equipment into the machine learning model to determine the target pressure signal's ventilation probability and ventilation time window, and controlling the ventilation equipment to perform automatic ventilation on the target individual according to the ventilation time window, includes:
Obtaining the ventilation compression ratio, target frequency, and ventilation threshold for different ventilation modes based on the mode setting parameters of the ventilation equipment;
Determining the target ventilation mode based on the target individual's compression frequency and the different modes' ventilation compression ratio, target frequency, and ventilation threshold;
Acquiring the ventilation compression phase under the target ventilation mode, and using a dynamic threshold method to match the ventilation compression phase based on the target individual's compression phase;
Determining the ventilation probability of the target pressure signal based on the matching results, and determining the ventilation time window threshold based on the relationship between the position of the ventilation compression phase and the target individual's compression phase;
Controlling the ventilation equipment to perform automatic ventilation on the target individual according to the ventilation time window threshold and the ventilation threshold.

The beneficial effects of the above technical solution are: it can both grasp the timing of ventilation for the target individual and control the amount of ventilation, further ensuring the safety of the target individual and the probability of successful resuscitation.

In an embodiment, this embodiment also discloses a ventilator suitable for the above cardiopulmonary resuscitation airway pressure automatic feedback ventilation method, the device includes: a ventilator body (1), a ventilation circuit (2), airflow and pressure detection equipment (3), a ventilation mask (4), and a pressure plate (5);
The pressure plate is used to perform artificial or mechanical chest compressions on the target individual;
The ventilation mask is used to cover the mouth and nose area of the target individual;
The airflow and pressure detection equipment is used to detect the airway pressure signal of the target individual's heart during compression;
The ventilator body is used to analyze the airway pressure signal, obtaining information about the target individual's cardiopulmonary resuscitation compressions, and based on this information, determining the ventilation time window, issuing ventilation commands, and performing ventilation operations;
The ventilation circuit is used to channel the ventilating agent from the ventilator body into the mouth and nose of the target individual.

The working principle and beneficial effects of the above technical solution have been explained in the method claims and are not repeated here.

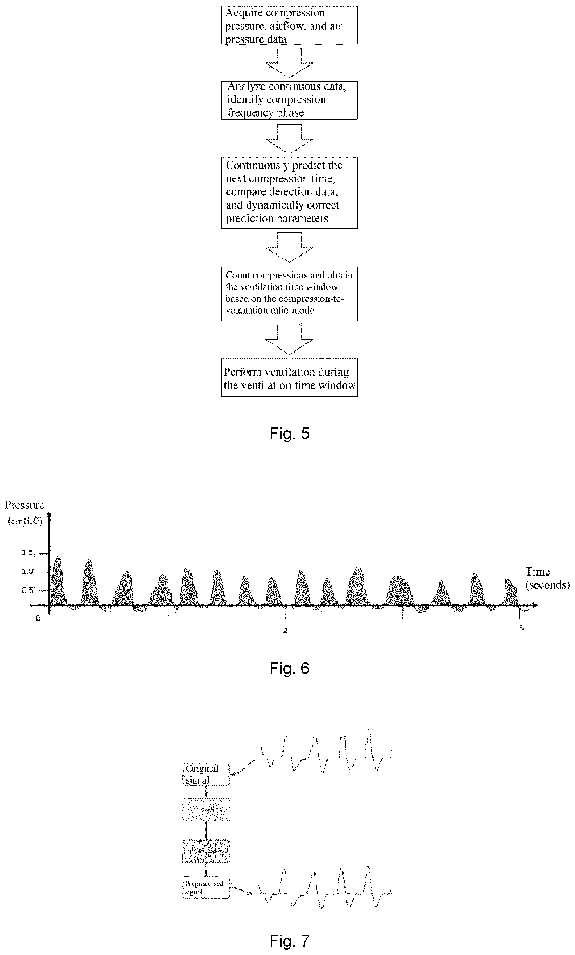

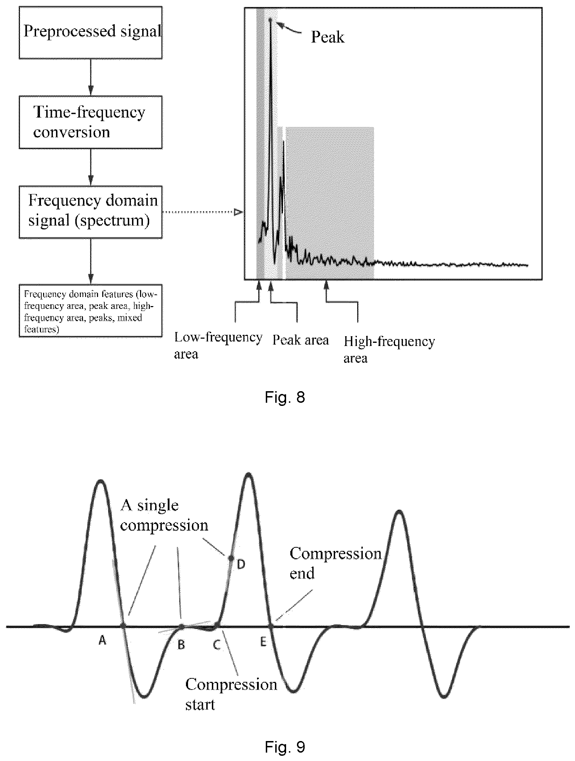

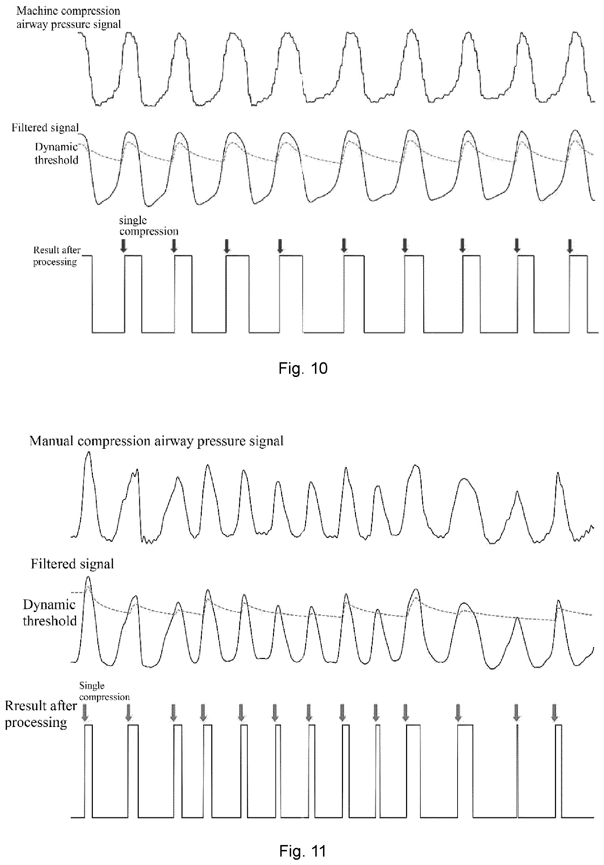

In an embodiment, as shown in Figure 5, by analyzing the compression pressure and airflow data, the airway pressure curve during the cardiac compression process is obtained, as shown in Figure 6. First, the signal is preprocessed, as shown in Figure 7, using a low-pass filter to remove high-frequency noise (such as equipment vibration, etc.) and other spurious signals (such as electromagnetic interference, etc.) contained in the original signal. Then, by eliminating the direct current signal, the baseline shift signal (such as the PEEP setting of the respiratory equipment, etc.) is removed to obtain a clean proximal pressure signal. Next, the signal is analyzed in the frequency domain and time domain, as shown in Figures 8 and 9. The time-domain and frequency-domain features obtained, combined with the mode settings of the ventilation equipment, are given to the machine learning model for judgment, to determine whether ventilation is needed, and to control the ventilation device to perform ventilation. The timing of ventilation for machine compression and manual compression is determined by a dynamic threshold method, as shown in Figures 10 and 11.

Those skilled in the technical field will readily think of other embodiments of this disclosure after considering the specification and practicing the disclosure herein. This application is intended to cover any variations, uses, or adaptations of the disclosure that follow the general principles of the disclosure and include means of common knowledge or customary skill in the art not disclosed herein.

It should be understood that the present disclosure is not limited to the precise structure that has been described above and illustrated in the accompanying drawings, and that various modifications and changes may be made without departing from its scope. The scope of the present disclosure is limited only by the appended claims.

## Claims

1. A ventilator, including: a ventilator body, a ventilation circuit, airflow and pressure detection equipment, a ventilation mask, and a pressure plate; wherein
the pressure plate is used to perform artificial or mechanical chest compressions on a target individual;
the ventilation mask is used to cover a mouth and nose area of the target individual;
the airflow and pressure detection equipment is used to detect an airway pressure signal of the target individual's heart during compression;
the ventilator body is used to analyze the airway pressure signal, obtain information about the target individual's cardiopulmonary resuscitation compressions, analyze to determine a ventilation time window based on the information, issue ventilation commands, and perform ventilation operations;
the ventilation circuit is used to channel a ventilating agent from the ventilator body into the mouth and nose of the target individual;
wherein the airflow and pressure detection equipment is configured for:
obtaining a current airway pressure signal when compressing the heart of the target individual at a preset sampling frequency;
the ventilator body is configured for:
dynamically correcting the current airway pressure signal based on body parameters of the target individual to obtain a target airway pressure signal;
continuously analyzing the target airway pressure signal to obtain a compression frequency, compression phase, and high-dimensional signal information for the target individual;
training a machine learning model with preset compression parameters and ventilation rule parameters; and
inputting the compression frequency, compression phase, and high-dimensional signal information for the target individual, along with mode setting parameters of the ventilator into the trained machine learning model to determine a ventilation probability and the ventilation time window of the target pressure signal, and controlling the ventilator to perform automatic ventilation on the target individual according to the ventilation time window;
wherein training the machine learning model with preset compression parameters and ventilation rule parameters includes:
generating compression pattern parameters based on a preset machine learning algorithm according to the preset compression parameters;
generating a first training sample based on the compression pattern parameters, setting model learning parameters and model output parameters based on the ventilation rule parameters; and
training the machine learning model with the first training sample based on the model learning parameters and model output parameters;
and wherein inputting the target individual's compression frequency, compression phase, and high-dimensional signal information, along with the mode setting parameters of the ventilator into the trained machine learning model to determine the target pressure signal's ventilation probability and ventilation time window, and controlling the ventilator to perform automatic ventilation on the target individual according to the ventilation time window, includes:
obtaining a ventilation compression ratio, target frequency, and ventilation threshold for respective ventilation modes based on the mode setting parameters of the ventilator, by the trained machine learning model;
determining a target ventilation mode based on the target individual's compression frequency and the respective modes' ventilation compression ratio, target frequency, and ventilation threshold, by the trained machine learning model;
acquiring a ventilation compression phase under the target ventilation mode, and using a dynamic threshold method to match the ventilation compression phase based on the target individual's compression phase, by the trained machine learning model;
determining the ventilation probability of the target pressure signal based on the matching results, and determining the ventilation time window threshold based on a relationship between a position of the ventilation compression phase and the target individual's compression phase, by the trained machine learning model; and
controlling the ventilator to perform automatic ventilation on the target individual according to the ventilation time window threshold and the ventilation threshold.

2. The ventilator according to claim 1, **characterized in that** the dynamic correction of the current airway pressure signal based on the body parameters of the target individual to obtain the target airway pressure signal includes:
detecting a compression force and speed when compressing the heart of the target individual;
setting the preset sampling frequency based on the compression force and speed, identifying the area around the mouth and nose of the target individual as a sampling area;
performing air pressure sampling on the target individual within the sampling area at the preset sampling frequency using a pressure sensor;
obtaining the current airway pressure signal when compressing the heart of the target individual based on the sampling results.

3. The ventilator according to claim 1, **characterized in that** before continuously analyzing the current airway pressure signal to obtain the compression frequency, compression phase, and high-dimensional signal information for the target individual, it further includes:
confirming the current airway pressure signal as an initial signal;
using a low-pass filter to remove high-frequency noise signals and other spurious signals from the initial signal, obtaining a first processed initial signal;
eliminating a baseline shift signal from the first processed initial signal by removing a direct current signal, obtaining a second processed initial signal;
generating a clean proximal pressure signal based on the second processed initial signal.

4. The ventilator according to claim 1, **characterized in that** the dynamic correction of the current airway pressure signal based on the body parameters of the target individual to obtain the target airway pressure signal includes:
determining current thoracic resonance parameters of the target individual based on the body parameters;
determining a dynamic correction factor based on the current thoracic resonance parameters and the preset collection parameters of the current airway pressure signal;
using the dynamic correction factor to dynamically correct the current airway pressure signal;
obtaining the target airway pressure signal based on the correction results.

5. The ventilator according to claim 3, **characterized in that** continuously analyzing the current airway pressure signal to obtain the compression frequency, compression phase, and high-dimensional signal information for the target individual includes:
analyzing the proximal pressure signal using a time-domain analysis algorithm to obtain a first analysis result;
analyzing the proximal pressure signal using a frequency-domain analysis algorithm to obtain a second analysis result;
determining the compression frequency, compression interval, and duration of a single compression for the target individual based on the first analysis result, and determining the compression phase based on the compression interval and duration of the single compression;
obtaining the frequency-domain characteristics of the signal based on the second analysis result, and performing a deep analysis of the signal frequency-domain characteristics to obtain the high-dimensional signal information.

6. The ventilator according to claim 5, **characterized in that** the analysis of the proximal pressure signal using the frequency-domain analysis algorithm to obtain the second analysis result includes:
using a window function and Fourier transform on the proximal pressure signal to perform a time-frequency conversion to obtain a spectral signal;
marking a peak of the spectral signal, capturing an area around the peak, as well as areas of the low-frequency and high-frequency regions:
obtaining regional signals corresponding to the area around the peak, and the areas of the low-frequency and high-frequency regions;
performing geometric calculations on the regional signals corresponding to the area around the peak, and the areas of the low-frequency and high-frequency regions using the frequency-domain analysis algorithm to obtain the second analysis result.

7. The ventilator according to claim 4, **characterized in that** the determination of the dynamic correction factor based on the current thoracic resonance parameters and the preset collection parameters of the current airway pressure signal includes:
determining set chest compression variation amplitude parameters based on the preset collection parameters of the current airway pressure signal;
determining an airway resistance index based on the chest compression variation amplitude parameters and the current thoracic resonance parameters;
generating a respiratory signal sample set using a respiratory mechanics model based on the airway resistance index;
determining morphological parameter values at various points in the target individual's airway based on the respiratory signal sample set;
determining a range of a stress tensor in the target individual's airway based on the morphological parameter values at various points in the airway;
acquiring characteristic parameters that maintain a steady-state performance of the target individual's airway, and obtaining airway performance indicators of the target individual based on the characteristic parameters;
determining a stress differentiation in the target individual's airway based on the airway performance indicators and the range of the airway's stress tensor;
determining standard data for the target individual's chest compression force and an error range of actual detection data based on the stress differentiation;
determining the dynamic correction factor for the current airway pressure signal based on the standard data for the target individual's chest compression force, the error range of actual detection data, and the detected pressure data corresponding to the current airway pressure signal.

## Patentansprüche

1. Beatmungsgerät, das beinhaltet: einen Beatmungsgerätekörper, einen Beatmungskreislauf, eine Luftstrom- und Druckerkennungsausrüstung, eine Beatmungsmaske und eine Druckplatte; wobei die Druckplatte verwendet wird, um künstliche oder mechanische Thoraxkompressionen bei einer Zielperson durchzuführen;
die Beatmungsmaske verwendet wird, um einen Mund- und Nasenbereich der Zielperson abzudecken;
die Luftstrom- und Druckerkennungsausrüstung verwendet wird, um ein Atemwegdrucksignal des Herzens der Zielperson während der Kompression zu erkennen;
der Beatmungsgerätekörper verwendet wird, um das Atemwegdrucksignal zu analysieren, Informationen über die Herz-Lungen-Beatmungskompressionen der Zielperson zu erhalten, zu analysieren, um basierend auf den Informationen ein Beatmungszeitfenster zu bestimmen, Beatmungsbefehle auszugeben und Beatmungsvorgänge durchzuführen;
der Beatmungskreislauf verwendet wird, um ein Beatmungsmittel von dem Beatmungsgerätekörper in den Mund und die Nase der Zielperson zu lenken;
wobei die Luftstrom- und Druckerkennungsausrüstung konfiguriert ist zum:
Erhalten eines aktuellen Atemwegdrucksignals beim Komprimieren des Herzens der Zielperson mit einer voreingestellten Abtastfrequenz;
der Beatmungsgerätekörper konfiguriert ist zum:
dynamischen Korrigieren des aktuellen Atemwegdrucksignals basierend auf Körperparametern der Zielperson, um ein Ziel-Atemwegdrucksignal zu erhalten;
kontinuierlichen Analysieren des Ziel-Atemwegdrucksignals, um eine Kompressionsfrequenz, Kompressionsphase und hochdimensionale Signalinformationen für die Zielperson zu erhalten;
Trainieren eines maschinellen Lernmodells mit voreingestellten Kompressionsparametern und Beatmungsregelparametern; und
Eingeben der Kompressionsfrequenz, der Kompressionsphase und hochdimensionalen Signalinformationen für die Zielperson zusammen mit Moduseinstellungsparametern des Beatmungsgeräts in das trainierte maschinelle Lernmodell, um eine Beatmungswahrscheinlichkeit und das Beatmungszeitfenster des Zieldrucksignals zu bestimmen, und Steuern des Beatmungsgeräts, um eine automatische Beatmung an der Zielperson gemäß dem Beatmungszeitfenster durchzuführen;
wobei Trainieren des maschinellen Lernmodells mit voreingestellten Kompressionsparametern und Beatmungsregelparametern beinhaltet:
Erzeugen von Kompressionsmusterparametern basierend auf einem voreingestellten maschinellen Lernalgorithmus gemäß den voreingestellten Kompressionsparametern;
Erzeugen einer ersten Trainingsabtastung basierend auf den Kompressionsmusterparametern, Einstellen von Modelllernparametern und Modellausgabeparametern basierend auf den Beatmungsregelparametern; und
Trainieren des maschinellen Lernmodells mit der ersten Trainingsabtastung basierend auf den Modelllernparametern und Modellausgabeparametern;
und wobei Eingeben der Kompressionsfrequenz, der Kompressionsphase und der hochdimensionalen Signalinformationen der Zielperson zusammen mit den Moduseinstellungsparametern des Beatmungsgeräts in das trainierte maschinelle Lernmodell, um die Beatmungswahrscheinlichkeit und das Beatmungszeitfenster des Zieldrucksignals zu bestimmen, und Steuern des Beatmungsgeräts, um eine automatische Beatmung der Zielperson gemäß dem Beatmungszeitfenster durchzuführen, beinhaltet:
Erhalten eines Beatmungskompressionsverhältnisses, einer Zielfrequenz und einer Beatmungsschwelle für jeweilige Beatmungsmodi basierend auf den Moduseinstellungsparametern des Beatmungsgeräts durch das trainierte maschinelle Lernmodell;
Bestimmen eines Zielbeatmungsmodus basierend auf der Kompressionsfrequenz der Zielperson und dem Kompressionsverhältnis, der Zielfrequenz und der Beatmungsschwelle der jeweiligen Modi durch das trainierte maschinelle Lernmodell;
Erfassen einer Beatmungskompressionsphase unter dem Zielbeatmungsmodus und Verwenden eines dynamischen Schwellenwertverfahrens, um die Beatmungskompressionsphase basierend auf der Kompressionsphase der Zielperson durch das trainierte maschinelle Lernmodell abzugleichen;
Bestimmen der Beatmungswahrscheinlichkeit des Zieldrucksignals basierend auf den abgeglichenen Ergebnissen und Bestimmen der Beatmungszeitfensterschwelle basierend auf einer Beziehung zwischen einer Position der Beatmungskompressionsphase und der Kompressionsphase der Zielperson durch das trainierte maschinelle Lernmodell; und
Steuern des Beatmungsgeräts, um eine automatische Beatmung der Zielperson gemäß der Beatmungszeitfensterschwelle und der Beatmungsschwelle durchzuführen.

2. Beatmungsgerät nach Anspruch 1, **dadurch gekennzeichnet, dass** die dynamische Korrektur des aktuellen Atemwegdrucksignals basierend auf den Körperparametern der Zielperson, um das Ziel-Atemwegdrucksignal zu erhalten, beinhaltet:
Erkennen einer Kompressionskraft und -geschwindigkeit beim Komprimieren des Herzens der Zielperson;
Einstellen der voreingestellten Abtastfrequenz basierend auf der Kompressionskraft und -geschwindigkeit, Identifizieren des Bereichs um Mund und Nase der Zielperson als Abtastbereich;
Durchführen einer Luftdruckabtastung an der Zielperson innerhalb des Abtastbereichs mit der voreingestellten Abtastfrequenz unter Verwendung eines Drucksensors; Erhalten des aktuellen Atemwegdrucksignals beim Komprimieren des Herzens der Zielperson basierend auf den Abtastergebnissen.

3. Beatmungsgerät nach Anspruch 1, **dadurch gekennzeichnet, dass** es vor dem kontinuierlichen Analysieren des aktuellen Atemwegdrucksignals, um die Kompressionsfrequenz, die Kompressionsphase und hochdimensionale Signalinformationen für die Zielperson zu erhalten, weiter beinhaltet:
Bestätigen des aktuellen Atemwegdrucksignals als Ausgangssignal;
Verwenden eines Tiefpassfilters, um hochfrequente Rauschsignale und andere Störsignale aus dem Ausgangssignal zu entfernen, um ein erstes verarbeitetes Ausgangssignal zu erhalten;
Beseitigen eines Grundlinienversatzsignals aus dem ersten verarbeiteten Ausgangssignal durch Entfernen eines Gleichstromsignals, Erhalten eines zweiten verarbeiteten Ausgangssignals;
Erzeugen eines sauberen proximalen Drucksignals basierend auf dem zweiten verarbeiteten Ausgangssignal.

4. Beatmungsgerät nach Anspruch 1, **dadurch gekennzeichnet, dass** die dynamische Korrektur des aktuellen Atemwegdrucksignals basierend auf den Körperparametern der Zielperson, um das Ziel-Atemwegdrucksignal zu erhalten, beinhaltet:
Bestimmen aktueller Thoraxresonanzparameter der Zielperson basierend auf den Körperparametern;
Bestimmen eines dynamischen Korrekturfaktors basierend auf den aktuellen Thoraxresonanzparametern und den voreingestellten Erhebungsparametern des aktuellen Atemwegdrucksignals;
Verwenden des dynamischen Korrekturfaktors, um das aktuelle Atemwegdrucksignal dynamisch zu korrigieren; Erhalten des Ziel-Atemwegdrucksignals basierend auf den Korrekturergebnissen.

5. Beatmungsgerät nach Anspruch 3, **dadurch gekennzeichnet, dass** das kontinuierliche Analysieren des aktuellen Atemwegdrucksignals, um die Kompressionsfrequenz, die Kompressionsphase und hochdimensionale Signalinformationen für die Zielperson zu erhalten, beinhaltet:
Analysieren des proximalen Drucksignals unter Verwendung eines Zeitbereichsanalysealgorithmus, um ein erstes Analyseergebnis zu erhalten;
Analysieren des proximalen Drucksignals mithilfe eines Frequenzbereichsanalysealgorithmus, um ein zweites Analyseergebnis zu erhalten;
Bestimmen der Kompressionsfrequenz, des Kompressionsintervalls und der Dauer einer einzelnen Kompression für die Zielperson basierend auf dem ersten Analyseergebnis und Bestimmen der Kompressionsphase basierend auf dem Kompressionsintervall und der Dauer der einzelnen Kompression;
Erhalten der Frequenz-Domänen-Eigenschaften des Signals basierend auf dem zweiten Analyseergebnis und Durchführen einer tiefgreifenden Analyse der Signalfrequenz-Domänen-Eigenschaften, um die hochdimensionalen Signalinformationen zu erhalten.

6. Beatmungsgerät nach Anspruch 5, **dadurch gekennzeichnet, dass** die Analyse des proximalen Drucksignals unter Verwendung des Frequenz-Domänen-Analysealgorithmus, um das zweite Analyseergebnis zu erhalten, beinhaltet:
Verwenden einer Fensterfunktion und Fourier-Transformation auf dem proximalen Drucksignal, um eine Zeit-FrequenzUmwandlung durchzuführen, um ein spektrales Signal zu erhalten;
Markieren eines Spitzenwerts des spektralen Signals, Erfassen eines Bereichs um den Spitzenwert herum, sowie von Bereichen des niederfrequenten und hochfrequenten Bereichs;
Erhalten regionaler Signale, die dem Bereich um den Spitzenwert und den Bereichen der niederfrequenten und hochfrequenten Regionen entsprechen;
Durchführen geometrischer Berechnungen auf den regionalen Signalen, die dem Bereich um den Spitzenwert herum entsprechen, und den Bereichen der niederfrequenten und hochfrequenten Regionen unter Verwendung des Frequenz-Domänen-Analysealgorithmus, um das zweite Analyseergebnis zu erhalten.

7. Beatmungsgerät nach Anspruch 4, **dadurch gekennzeichnet, dass** die Bestimmung des dynamischen Korrekturfaktors basierend auf den aktuellen Thoraxresonanzparametern und den voreingestellten Erhebungsparametern des aktuellen Atemwegdrucksignals beinhaltet:
Bestimmen von eingestellten Amplitudenparametern der Brustkompressionsvariation basierend auf den voreingestellten Erhebungsparametern des aktuellen Atemwegdrucksignals;
Bestimmen eines Atemwegswiderstandsindex basierend auf den Amplitudenparametern der Brustkompressionsvariation und den aktuellen Thoraxresonanzparametern;
Erzeugen eines Atemsignalabtastsatzes unter Verwendung eines Modells der Atemmechanik basierend auf dem Atemwegswiderstandsindex;
Bestimmen morphologischer Parameterwerte an verschiedenen Stellen in den Atemwegen der Zielperson basierend auf dem Atemsignalabtastsatz;
Bestimmen eines Bereichs eines Spannungstensors in den Atemwegen der Zielperson basierend auf den morphologischen Parameterwerten an verschiedenen Stellen in den Atemwegen; Erfassen charakteristischer Parameter, die eine stabile Leistung der Atemwege der Zielperson aufrechterhalten, und Erhalten von Atemwegsleistungsindikatoren der Zielperson basierend auf den charakteristischen Parametern; Bestimmen einer Spannungsdifferenzierung in den Atemwegen der Zielperson basierend auf den Atemwegsleistungsindikatoren und dem Bereich des Spannungssensors der Atemwege;
Bestimmen von Standarddaten für die Brustkompressionskraft der Zielperson und eines Fehlerbereichs von tatsächlichen Erkennungsdaten basierend auf der Spannungsdifferenzierung; Bestimmen des dynamischen Korrekturfaktors für das aktuelle Atemwegdrucksignal basierend auf den Standarddaten für die Brustkompressionskraft der Zielperson, dem Fehlerbereich der tatsächlichen Erkennungsdaten und den erkannten Druckdaten, die dem aktuellen Atemwegdrucksignal entsprechen.

## Revendications

1. Ventilateur, incluant : un corps de ventilateur, un circuit de ventilation, un équipement de détection de débit d'air et de pression, un masque de ventilation et une plaque de pression ; dans lequel
la plaque de pression est utilisée pour réaliser des compressions artificielles ou mécaniques de la poitrine sur un individu cible ;
le masque de ventilation est utilisé pour recouvrir une zone de la bouche et du nez de l'individu cible ; l'équipement de détection de débit d'air et de pression est utilisé pour détecter un signal de pression des voies aériennes du cœur de l'individu cible pendant la compression ;
le corps de ventilateur est utilisé pour analyser le signal de pression des voies aériennes, obtenir des informations sur les compressions de réanimation cardiopulmonaire de l'individu cible, analyser pour déterminer une fenêtre de temps de ventilation sur la base des informations, émettre des instructions de ventilation et réaliser des opérations de ventilation ;
le circuit de ventilation est utilisé pour canaliser un agent de ventilation à partir du corps de ventilateur dans la bouche et le nez de l'individu cible ;
dans lequel l'équipement de détection de débit d'air et de pression est configuré pour :
l'obtention d'un signal de pression des voies aériennes actuel lors de la compression du cœur de l'individu cible à une fréquence d'échantillonnage prédéfinie ;
le corps de ventilateur est configuré pour :
la correction dynamique du signal de pression des voies aériennes actuel sur la base de paramètres corporels de l'individu cible pour obtenir un signal de pression des voies aériennes cible ;
l'analyse continue du signal de pression des voies aériennes cible pour obtenir une fréquence de compression, une phase de compression et des informations de signal hautement dimensionnel pour l'individu cible ; l'entraînement d'un modèle d'apprentissage automatique avec des paramètres de compression prédéfinis et des paramètres de règle de ventilation ; et
l'entrée des informations de fréquence de compression, de phase de compression et de signal hautement dimensionnel pour l'individu cible, ainsi que des paramètres de réglage de mode du ventilateur dans le modèle d'apprentissage automatique entraîné pour déterminer une probabilité de ventilation et la fenêtre de temps de ventilation du signal de pression cible, et la commande du ventilateur pour qu'il réalise une ventilation automatique sur l'individu cible en fonction de la fenêtre de temps de ventilation ;
dans lequel l'entraînement du modèle d'apprentissage automatique avec des paramètres de compression prédéfinis et des paramètres de règle de ventilation inclut :
la génération de paramètres de modèle de compression sur la base d'un algorithme d'apprentissage automatique prédéfini en fonction des paramètres de compression prédéfinis ;
la génération d'un premier échantillon d'entraînement sur la base des paramètres de modèle de compression, le réglage des paramètres d'apprentissage de modèle et des paramètres de sortie de modèle sur la base des paramètres de règle de ventilation ; et
l'entraînement du modèle d'apprentissage automatique avec le premier échantillon d'entraînement sur la base des paramètres d'apprentissage de modèle et des paramètres de sortie de modèle ;
et dans lequel l'entrée de la fréquence de compression, de la phase de compression et des informations de signal hautement dimensionnel de l'individu cible, ainsi que des paramètres de réglage de mode du ventilateur dans le modèle d'apprentissage automatique entraîné pour déterminer la probabilité de ventilation et la fenêtre de temps de ventilation du signal de pression cible, et la commande du ventilateur pour qu'il réalise une ventilation automatique sur l'individu cible en fonction de la fenêtre de temps de ventilation, inclut :
l'obtention d'un rapport de compression de ventilation, d'une fréquence cible et d'un seuil de ventilation pour les modes de ventilation respectifs sur la base des paramètres de réglage de mode du ventilateur, par le modèle d'apprentissage automatique entraîné ;
la détermination d'un mode de ventilation cible sur la base de la fréquence de compression de l'individu cible et du rapport de compression de ventilation, de la fréquence cible et du seuil de ventilation des modes respectifs, par le modèle d'apprentissage automatique entraîné ; l'acquisition d'une phase de compression de ventilation sous le mode de ventilation cible, et l'utilisation d'une méthode de seuil dynamique pour faire correspondre la phase de compression de ventilation sur la base de la phase de compression de l'individu cible, par le modèle d'apprentissage automatique entraîné ;
la détermination de la probabilité de ventilation du signal de pression cible sur la base des résultats correspondants, et la détermination du seuil de fenêtre de temps de ventilation sur la base d'une relation entre une position de la phase de compression de ventilation et la phase de compression de l'individu cible, par le modèle d'apprentissage automatique entraîné ; et
la commande du ventilateur pour qu'il réalise une ventilation automatique sur l'individu cible en fonction du seuil de fenêtre de temps de ventilation et du seuil de ventilation.

2. Ventilateur selon la revendication 1, **caractérisé en ce que** la correction dynamique du signal de pression des voies aériennes actuel sur la base des paramètres corporels de l'individu cible pour obtenir le signal de pression des voies aériennes cible inclut :
la détection d'une force et d'une vitesse de compression lors de la compression du cœur de l'individu cible ;
le réglage de la fréquence d'échantillonnage prédéfinie sur la base de la force et de la vitesse de compression, l'identification de la zone autour de la bouche et du nez de l'individu cible en tant que zone d'échantillonnage ; la réalisation d'un échantillonnage de pression d'air sur l'individu cible au sein de la zone d'échantillonnage à la fréquence d'échantillonnage prédéfinie à l'aide d'un capteur de pression ;
l'obtention du signal de pression des voies aériennes actuel lors de la compression du cœur de l'individu cible sur la base des résultats d'échantillonnage.

3. Ventilateur selon la revendication 1, **caractérisé en ce qu'**avant d'analyser en continu le signal de pression des voies aériennes actuel pour obtenir la fréquence de compression, la phase de compression et des informations de signal hautement dimensionnel pour l'individu cible, il inclut en outre :
la confirmation du signal de pression des voies aériennes actuel en tant que signal initial ;
l'utilisation d'un filtre passe-bas pour supprimer des signaux de bruit haute fréquence et d'autres signaux parasites du signal initial, l'obtention d'un premier signal initial traité ;
l'élimination d'un signal de décalage de ligne de base à partir du premier signal initial traité par suppression d'un signal de courant continu, l'obtention d'un second signal initial traité ;
la génération d'un signal de pression proximal propre sur la base du second signal initial traité.

4. Ventilateur selon la revendication 1, **caractérisé en ce que** la correction dynamique du signal de pression des voies aériennes actuel sur la base des paramètres corporels de l'individu cible pour obtenir le signal de pression des voies aériennes cible inclut :
la détermination de paramètres de résonance thoracique actuels de l'individu cible sur la base des paramètres corporels ;
la détermination d'un facteur de correction dynamique sur la base des paramètres de résonance thoracique actuels et des paramètres de collecte prédéfinis du signal de pression des voies aériennes actuel ;
l'utilisation du facteur de correction dynamique pour corriger dynamiquement le signal de pression des voies aériennes actuel ;
l'obtention du signal de pression des voies aériennes cible sur la base des résultats de correction.

5. Ventilateur selon la revendication 3, **caractérisé en ce que** l'analyse continue du signal de pression des voies aériennes actuel pour obtenir la fréquence de compression, la phase de compression et des informations de signal hautement dimensionnel pour l'individu cible inclut :
l'analyse du signal de pression proximal à l'aide d'un algorithme d'analyse de domaine temporel pour obtenir un premier résultat d'analyse ;
l'analyse du signal de pression proximal à l'aide d'un algorithme d'analyse de domaine fréquentiel pour obtenir un second résultat d'analyse ;
la détermination de la fréquence de compression, de l'intervalle de compression et de la durée d'une seule compression pour l'individu cible sur la base du premier résultat d'analyse, et la détermination de la phase de compression sur la base de l'intervalle de compression et de la durée de la seule compression ;
l'obtention des caractéristiques de domaine fréquentiel du signal sur la base du second résultat d'analyse, et la réalisation d'une analyse approfondie des caractéristiques de domaine fréquentiel de signal pour obtenir les informations de signal hautement dimensionnel.

6. Ventilateur selon la revendication 5, **caractérisé en ce que** l'analyse du signal de pression proximal à l'aide de l'algorithme d'analyse de domaine fréquentiel pour obtenir le second résultat d'analyse inclut :
l'utilisation d'une fonction fenêtre et d'une transformée de Fourier sur le signal de pression proximal pour réaliser une conversion temps-fréquence afin d'obtenir un signal spectral ;
le marquage d'un pic du signal spectral, la capture d'une zone autour du pic, ainsi que de zones des régions basse fréquence et haute fréquence ;
l'obtention de signaux régionaux correspondant à la zone autour du pic, et aux zones des régions basse fréquence et haute fréquence ;
la réalisation de calculs géométriques sur les signaux régionaux correspondant à la zone autour du pic, et aux zones des régions basse fréquence et haute fréquence à l'aide de l'algorithme d'analyse de domaine fréquentiel afin d'obtenir le second résultat d'analyse.

7. Ventilateur selon la revendication 4, **caractérisé en ce que** la détermination du facteur de correction dynamique sur la base des paramètres de résonance thoracique actuels et des paramètres de collecte prédéfinis du signal de pression des voies aériennes actuel inclut :
la détermination de paramètres d'amplitude de variation de compression de poitrine réglés sur la base des paramètres de collecte prédéfinis du signal de pression des voies aériennes actuel ;
la détermination d'un indice de résistance des voies aériennes sur la base des paramètres d'amplitude de variation de compression de poitrine et des paramètres de résonance thoracique actuels ;
la génération d'un ensemble d'échantillons de signaux respiratoires à l'aide d'un modèle de mécanique respiratoire sur la base de l'indice de résistance des voies aériennes ;
la détermination de valeurs de paramètres morphologiques à divers points dans les voies aériennes de l'individu cible sur la base de l'ensemble d'échantillons de signaux respiratoires ;
la détermination d'une plage d'un tenseur de contrainte dans les voies aériennes de l'individu cible sur la base des valeurs de paramètres morphologiques à divers points dans les voies aériennes ;
l'acquisition de paramètres caractéristiques qui maintiennent une performance à l'état stable des voies aériennes de l'individu cible, et l'obtention d'indicateurs de performance des voies aériennes de l'individu cible sur la base des paramètres caractéristiques ;
la détermination d'une différenciation de contrainte dans les voies aériennes de l'individu cible sur la base des indicateurs de performance des voies aériennes et de la plage du tenseur de contrainte des voies aériennes ;
la détermination de données standard pour la force de compression de poitrine de l'individu cible et d'une plage d'erreur de données de détection réelles sur la base de la différenciation de contrainte ;
la détermination du facteur de correction dynamique pour le signal de pression des voies aériennes actuel sur la base des données standard pour la force de compression de poitrine de l'individu cible, de la plage d'erreur des données de détection réelles et des données de pression détectées correspondant au signal de pression des voies aériennes actuel.
